# EUROPEAN PATENT SPECIFICATION

(11) **EP 3 228 625 B1**
(45) Date of publication and mention of the grant of the patent: **20.02.2019**
(21) Application number: 15877635.1
(22) Date of filing: 28.10.2015
(51) Int. Cl.: C07K 1/14, C07K 14/435, C07K 1/12

(54) **PREPARATION METHOD FOR WATER-SOLUBLE CONCHIOLIN AND ACID-SOLUBLE CONCHIOLIN**
VERFAHREN ZUR HERSTELLUNG VON WASSERLÖSLICHES CONCHIOLIN UND SÄURELÖSLICHES CONCHIOLIN
PROCÉDÉ DE PRÉPARATION DE CONCHYOLINE SOLUBLE DANS L'EAU ET CONCHYOLINE SOLUBLE DANS L'ACIDE

(30) Priority: 15.01.2015 CN 201510020372
(43) Date of publication of application: 11.10.2017
(73) Proprietor: Osmun Biological Co., Ltd., Huzhou, Zhejiang 313200 (CN)
(72) Inventor: YANG, Anquan, Huzhou Zhejiang 313200 (CN); WANG, Jing, Huzhou Zhejiang 313200 (CN); ZHANG, Lihua, Huzhou Zhejiang 313200 (CN)
(74) Representative: Sun, Yiming
(86) International application number: PCT/CN2015/093020
(87) International publication number: WO 2016/112722

(56) References cited:
- CN-A- 1 526 326
- CN-A- 101 104 001
- CN-A- 101 381 400
- CN-A- 101 611 862
- CN-A- 103 333 222
- CN-A- 103 804 468
- CN-A- 104 558 137
- CN-B- 103 333 222

## Description

### BACKGROUND OF THE INVENTION

### Field of the Invention

The present invention relates to the field of pearl extract production, in particular, relates to a pearl protein preparation method. Also disclosed are a water-soluble pearl protein and acid-soluble pearl protein obtained by adopting this method.

### Background Technology

Currently, in the application of pearl powder, numerous problems such as grain size, potential safety hazard, poor liquidity, water insolubility and difficulty in the release of the functional components have been encountered, which directly affect the application fields of pearl powder. To solve the problems above, water-soluble pearl powder and pearl hydrolysate have been developed. However, during these processes, we only conduct simple compounding of the original components or extensive hydrolysis, while no research was performed on the functions of each component therein, and fine separation and purification were rarely conducted.

The existing pearl extracts are generally produced by adopting enzymolysis method. For example, the Chinese invention patent (application number: 01139157.X; application date: December 19, 2001) discloses a pearl powder preparation method by biological enzymolysis. The key lies in the use of lactic acid solution as a solvent for dissolving the pearl, adjusting the pH of the solution to near neutral, readjusting it to a meta-acid by using acid, and then adding appropriate amounts of biological active enzyme to realize catalytic decomposition of the protein to obtain an enzymatic pearl glue solution, adopting spray drying to realize rapid drying of the enzymatic pearl glue solution, curing and pelletizing, and obtaining the superfine enzymatic pearl powder. The pearl powder prepared reserves effective components such as various amino acids and microelements of the pearl, and the calcium carbonate of the pearl are translated into active calcium that is easily absorbed by the human body, and the keratin protein insoluble in gastric acid are converted to free amino acids that are easily absorbed by enzymolysis.

The Chinese invention patent (application number: 200710141569.X; application date: August 07, 2007) discloses a pearl bioactive peptide preparation method as follows: taking out pearl from fresh pearl shell, mixing pearl with deionized water, adjusting PH of the mixed solution to a meta-acid with phosphate buffer solution, and then using serrapeptass to realize an enzymatic hydrolysis reaction with pearl; obtaining pearl and a clear solution by filtering separation with filters, boiling the reaction solution for enzyme deactivation; and then performing spray drying of the obtained solution to obtain a pearl bioactive peptide.

The Chinese invention patent (application number: 200910114409.5; application date: September 18, 2009) discloses a pearl hydrolysate preparation method, with steps as follows: (1) putting pearl powder in hydrochloric acid water solution, stirring it until full reaction, adjusting pH of this mixture to neutral with ammonium hydroxide, and performing centrifugation to obtain sediment A; (2) dissolving the sediment A in purified water, adjusting the pH value to 7.5∼8.5 with ammonium hydroxide, and performing centrifugation to obtain sediment B; (3) dissolving the sediment B in purified water, realizing enzymolysis by adding in an enzyme, performing centrifugation to the enzymatic hydrolysate, and obtaining a supernatant and sediment C; wherein the named enzyme is a mixture of snail protease and papain, with an addition quantity of 0.3∼1.05% based on a weight of sediment B; (4) dissolving the sediment C in purified water, adding in an enzyme to perform secondary enzymolysis, performing centrifugation, and collecting the supernatant; wherein the named enzyme is a mixture of trypsin, papain and neutral protease, with an addition quantity of 0.2∼0.45% based on a weight of sediment B; (5) combining the two supernatants, boiling it, filtering by using a nanofiltration membrane with a pore diameter 1∼3nm, and obtaining the pearl hydrolysate.

The aforementioned methods adopt enzymolysis, which can be used to obtain amino acids and polypeptide with low molecular weight, however, enzymolysis can destroy some activated protein substances and simultaneously cause loss of the active substances.

The Chinese invention patent (application number: 201410055730.1; application date: February 19, 2014) discloses a nanoscale pearl protein preparation method as follows: grinding pearl powder into micro pearl powder having mean grain size of 1∼ 4 micrometers, performing acidolysis and filtering, washing the filtered residue with purified water to obtain coarse pearl protein, adding a dispersing agent to the coarse pearl protein and grinding it, and performing ultrasonic oscillation during the process; concentrating the solution obtained after grinding, heating it for sterilization, and saving it to obtain a nanoscale pearl protein. In this method, a dispersing agent is added, which is hard to be eliminated in subsequent processing steps, hence limiting the application of the product. Other patents describing the preparation of pearl extract powder include CN103333222B, which describes an ultrasonic method for pearl protein extraction, and CN101611862, CN1526326 and CN101104001, which all describe high pressure homogenization methods for the extraction of pearl proteins.

### SUMMARY OF THE INVENTION

### Technical Problem

To solve the aforementioned technical problem, this invention aims to provide a pearl protein preparation method as follows: a nanoscale pearl powder is used, desalination is performed after hydrolysis and acidolysis, a water-soluble pearl protein and an acid-soluble pearl protein are obtained respectively, which provides raw materials for pearl skin care products and health care products.

### Technical Solution

To realize the purpose above, this invention adopts the technical solution below:
A pearl protein preparation method, the method includes the following steps:
1) Grinding pearls into a nanoscale pearl powder;
2) Performing ultra-high pressure homogenization to the nanoscale pearl powder using deionized water bath at 40∼80°C for 0.2∼1h, proceed with centrifugation at low temperatures of 0∼8°C to obtain a water-soluble pearl extract a and sediments, rinse the sediments with ethanol solution, and then conduct the next operation;
3) Dissolving the sediments obtained above in weak acid at temperatures of 30∼ 50°C, stirring at a low speed of 80-120 rpm, and reacting for 0.5∼3h, performing centrifugation at low temperatures of 0∼8°C, and obtaining a supernatant being an acid-soluble pearl extract b;
4) Performing desalination of the water-soluble pearl extract a and the acid-soluble pearl extract b respectively;
5) Freeze-drying the water-soluble pearl extract a and the acid-soluble pearl extract b after desalination, and obtaining a water-soluble pearl protein and an acid-soluble pearl protein respectively.

Preferably, a grain size of the described nanoscale pearl powder is 10∼100nm.

Preferably, the described weak acid adopts edible acetic acid, citric acid or carbonic acid.

Preferably, the described desalination adopts dialysis desalination: the desalination is performed by using a dialysis bag with molecular weight of 3000∼5000Da at a 2∼10°C environment for 3-5 days, wherein 1 volume of sample corresponds with 10-20 volumes of distilled water, and water needs to be changed 1-2 times each day.

Preferably, the described desalination adopts chromatography desalination: using Sephadex G-15 for column packing, start to collect after first desalination and sample adding, collect once every 1-2 minutes, gather several samples, add in little amounts of ammonium sulfate respectively, determine if sediments are present, verify which of the samples have salt out, and collect solutions before this time point which is to be treated as a protein solution after desalination.

As to a water-soluble pearl protein prepared by adopting the method stated in this invention, the water-soluble pearl protein has a main molecular weight distribution of less than 15kd. "Main" of this invention refers to over 90% of the substances in this protein.

As to an acid-soluble pearl protein prepared by adopting the method stated in this invention, the acid-soluble pearl protein has a main molecular weight distribution of 14kd∼50kd. "Main" of this invention refers to over 90% of the substances in this protein.

### Advantageous Effects

As this invention adopts the aforementioned technical solution, which uses nanoscale pearl powder, performs desalination after hydrolysis and acidolysis to obtain water-soluble pearl protein and acid-soluble pearl protein respectively, raw materials for pearl skin care products and health care products can be provided. Specifically, acid-soluble pearl protein possesses strong antioxidant capacity, can promote fibroblast growth, simultaneously endorse the secretion of collagen, is favorable for wound healing, and has powerful potential in the field of cosmetic treatment. On the other hand, water-soluble pearl protein can obtain favorable effect in aspects of skin whitening and moisturizing.

### BRIEF DESCRIPTION OF THE DRAWINGS

FIG. 1 is a molecular weight distribution diagram of an acid-soluble pearl protein.
FIG. 2 is a molecular weight distribution diagram of a water-soluble pearl protein.
FIG. 3 is an influence diagram of different pearl proteins regarding fibroblast growth.
FIG. 4 is an influence diagram of different pearl proteins regarding DPPH free radical scavenging rate.
FIG. 5 is an influence diagram of an acid-soluble pearl protein regarding collagen secretion of fibroblast.

### DESCRIPTION OF THE EMBODIMENTS

### Example 1

A pearl protein preparation method, the method includes the following steps:
1) Grinding pearls into a nanoscale pearl powder (10∼100nm);
2) Performing ultra-high pressure homogenization to the nanoscale pearl powder using 10-times-mass of a deionized water bath at 60°C for 0.5h, proceed with centrifugation at a low temperature of 4°C to obtain a water-soluble pearl extract a and sediments, rinse the sediments with ethanol solution for 3 times, and then conduct the next operation;
3) Dissolving the sediments obtained above in weak acid under intermediate temperatures and vacuum (40°C, 101325∼1333pa), stirring at a low speed (100rpm), and react for 2h; perform centrifugation at a low temperature of 4°C, and obtain a supernatant being an acid-soluble pearl extract b;
4) Perform desalination to the pearl extracts a and b by adopting the two methods below;
   ① Dialysis desalination: the desalination is performed by using a dialysis bag with molecular weight of 3500Da at a 4∼7°C environment for 4 days, wherein 1 volume of sample corresponds with 10-15 volumes of distilled water, and water needs to be changed 1 time each day.
   Or ② chromatography desalination: Sephadex G-15 was used for column packing, start to collect after first desalination and sample adding, collect once every 1 minute, gather 15 samples, add in little amounts of ammonium sulfate respectively, determine whether sediments are present, verify which of the samples have salt out in this step, and collect solutions before this time point which is to be treated as a protein solution after desalination; finally, a water-soluble pearl protein a and an acid-soluble pearl protein b can be obtained correspondingly;
5) Collect solutions a and b by vacuum freeze-drying, and a water-soluble pearl protein and an acid-soluble pearl protein can be obtained respectively.

### Example 2

Molecular weight detection of different pearl proteins, using test method: SDS-PAGE detection method
a) Take 20µl of a 1 times diluted water-soluble pearl protein and acid-soluble pearl protein respectively, add in 5µl of a 5-times concentrated sample buffer solution respectively, and boil at an 80°C water bath kettle for 5min;
b) Prepare SDS-PAGE gel according to the recipe above, install an electrophoresis apparatus, and add electrode buffer solution in the trough;
c) Add samples in sample adding holes in a particular sequence, wherein an amount of added sample is selected based on a difference between a gap thickness of the SDS-PAGE electrophoresis glass plates. In general, for 0.75mm gap with 15 holes, a sample adding amount is less than 10 µL /hole, for 1 mm gap with 10 holes, a sample adding amount is less than 20µL/hole. The sample adding amount may be determined according to practical experience;
d) Open power supply, adjust voltage to 80v (generally takes about 15 min), and adjust voltage to 120v after the sample passes through the compressed gel, maintain the voltage until the electrophoresis of the sample reaches the bottom of the gel;
e) Carefully remove the gel and placing it into a container, add in staining fluid, and shake for 2-3h with a shaker;
f) Pour out the staining fluid after the stripe becomes clear, then add in a destaining solution, and leave overnight;
g) Pour away the destaining solution, use Quality One, or a corresponding imaging system to take a picture, then analyze and estimate the protein concentration.

### Test results

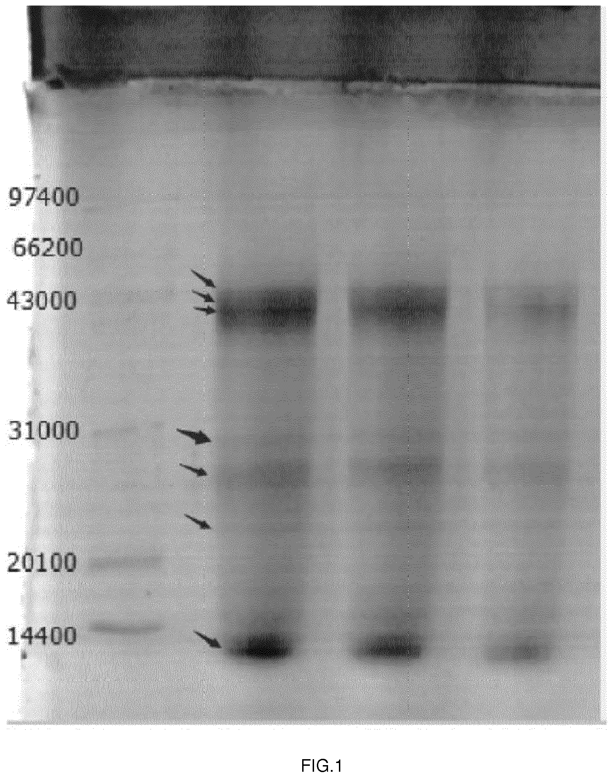

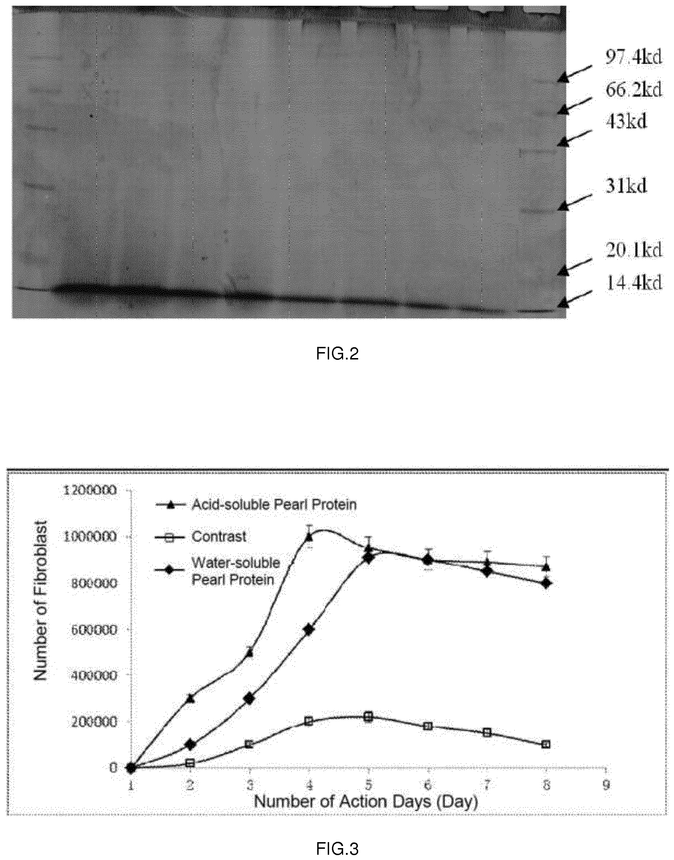

FIG. 1 is the acid-soluble pearl protein, the molecular weight distributions are in the ranges of 14.4, 31 and 43kd, which belongs to a macromolecule protein. FIG. 2 is a molecular weight measurement diagram of the water-soluble pearl protein, the molecular weight distribution mainly concentrates at 14.4 or even at lower molecular weight ranges, which belongs to a small molecular weight protein.

### Example 3

### 1. Research on the influence of different pearl proteins on fibroblast growth

### a) Test method

### Cell growth curve diagram test

Influence of different pearl proteins on fibroblast proliferation can be reflected by detecting the number of cells in an 8-day cultivation process. The acid-soluble pearl protein and the water-soluble pearl protein were each prepared into a series of 15.6 µg/ml medium, and 1% calf serum was added; take 1% of calf serum culture medium as blank contrast. The number of cells were counted using cell counting plates each day.

### b) Test results

As shown in FIG. 3, the acid-soluble pearl protein can promote cell growth, and an obvious difference is observed as compared with the blank contrast containing 1% calf serum. At the same time, the water-soluble pearl protein also has the function of significantly promoting the effective growth of fibroblast. However, the action time is slightly lagging as compared with the acid-soluble protein. Generally speaking, they both have the function of promoting fibroblast growth rate.

### 2. Research on DDPH free radical scavenging abilities of different pearl proteins

### a) Test method

### DPPH free radical inhibiting ability

Test steps: preparing DPPH95% ethanol solution with a concentration of 2×10⁻⁴mol/L, and protecting it from light. Taking 2mlDPPH solution, and adding in 2ml of acid-soluble pearl protein solution or water-soluble pearl protein solution with different concentrations respectively, mixing it well, reacting it for 30min at 25°C, measuring the absorption value at 517nm (the maximum absorption wavelength), calculating the scavenging rate, and taking it as reference.

Calculation formula of DPPH free radical scavenging activity: P=[(1-(Aᵢ-Aⱼ) /Ac)]×100%

In the formula: Aᵢ= absorbance of 2mlDPPH solution +2ml sample solution.
Aⱼ= absorbance of 2ml sample solution+2ml solvent.
A_{c}= absorbance of 2mlDPPH solution+2ml solvent.

### b) Test results

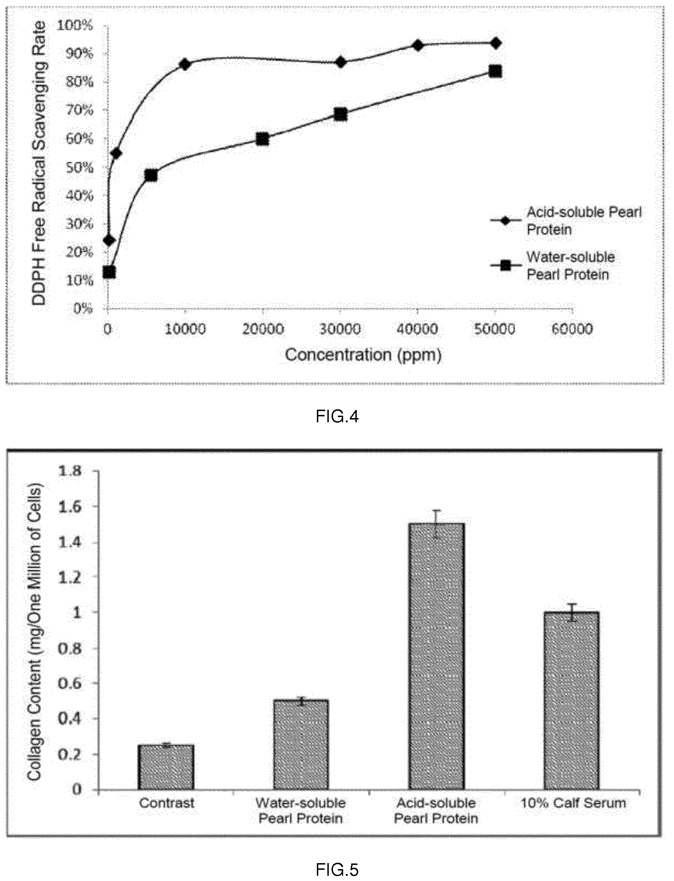

As shown in FIG. 4, the DPPH free radical scavenging ability of acid-soluble pearl protein is obviously higher than that of water-soluble pearl protein, which illustrates the stronger antioxidant ability of the acid-soluble pearl protein.

### 3. Influence of different pearl proteins on collagen generation of cells

### a) Test method

### Hydroxyproline test

The collagen products of fibroblast can be evaluated by quantifying the amount of hydroxyproline. Fibroblasts are inoculated in a 24-hole plate, and treated in the presence of an acid-soluble pearl protein with a concentration of 15.6µg/ml for 72 hours. The 1% calf serum culture medium and 10% calf serum culture medium are used as blank contrast and positive contrast respectively. Chloramine T method is used for detecting the content of hydroxyproline. The data refers to microgram of the collagen in 10⁶ cells, assuming that there are 13.5% hydroxyprolines contained in collagen.

### b) Test result

As shown in FIG. 5, by comparing the blank contrast with the higher-content calf serum contrast, the acid-soluble pearl protein possesses significant function of enhancing collagen secretion. As compared with the blank contrast, the water-soluble pearl protein can promote collagen secretion substantially, but its function is lower than the function of the 10% calf serum. Thus, it is visible that acid-soluble pearl protein possesses more advantages in fibroblast promotion.

## Claims

1. A pearl protein preparation method, the method is **characterized by** the following steps:
1) grinding pearls into a nanoscale pearl powder;
2) performing ultra-high pressure homogenization to the nanoscale pearl powder using deionized water bath at 40 ∼ 80°C for 0.2∼1h, proceed with centrifugation at low temperatures of 0∼8°C to obtain a water-soluble pearl extract a and sediments, and rinsing the sediments with an ethanol solution for 2-4 times;
3) dissolving the sediments obtained above in weak acid at temperatures of 30 ∼ 50°C under vacuum, stirring at a low speed of 80-120 rpm, and reacting for 0.5 ∼ 3h, performing centrifugation at low temperature of 0∼8°C, and obtaining a supernatant being an acid-soluble pearl extract b;
4) performing desalination of the water-soluble pearl extract a and the acid-soluble pearl extract b respectively;
5) freeze-drying the water-soluble pearl extract a and the acid-soluble pearl extract b after desalination, and obtaining a water-soluble pearl protein and an acid-soluble pearl protein respectively.

2. The pearl protein preparation method according to claim 1, **characterized by** that a grain size of the nanoscale pearl powder is 10∼100nm.

3. The pearl protein preparation method according to claim 1, **characterized by** that the weak acid adopts edible acetic acid, citric acid or carbonic acid.

4. The pearl protein preparation method according to claim 1, **characterized by** that the desalination adopts dialysis desalination: the desalination is performed by using a dialysis bag with molecular weight of 3000∼5000Da at a 2∼10°C environment for 3-5 days, wherein 1 volume of sample corresponds with 10-20 volumes of distilled water, and water needs to be changed 1-2 times each day.

5. The pearl protein preparation method according to claim 1, **characterized by** that the desalination adopts chromatography desalination: using Sephadex G-15 for column packing, start to collect after first desalination and sample adding, collect once every 1-2 minutes, gather several samples, add in little amounts of ammonium sulfate respectively, determine if sediments are present, verify which of the samples have salt out, and collect solutions before this time point which is to be treated as a protein solution after desalination.

## Patentansprüche

1. Verfahren zur Herstellung von Perlenprotein, das sich durch die folgenden Schritte kennzeichnet:
• 1) Mahlen von Perlen zu einem nanoskaligen Perlenpulver;
• 2) Durchführen einer Ultrahochdruck-Homogenisierung des nanoskaligen Perlenpulvers unter Verwendung eines entionisierten Wasserbades bei 40∼80°C über 0.2 ∼ 1h, Fortfahren mit Zentrifugation bei niedrigen Temperaturen von 0∼8°C, um einen wasserlöslichen Perlenextrakt a und Sedimente zu erhalten, und Spülen der Sedimente mit einer Ethanollösung 2-4 Mal;
• 3) Lösen der oben erhaltenen Sedimente in schwacher Säure bei Temperaturen von 30∼50°C unter Vakuum, Rühren bei einer niedrigen Geschwindigkeit von 80-120 U/min und Umsetzen über 0.5∼3 Stunden, Durchführen einer Zentrifugation bei einer niedrigen Temperatur von 0∼8°C und Erhalten eines Überstands, der ein säurelöslicher Perlenextrakt b ist;
• 4) Durchführen jeweils einer Entsalzung des wasserlöslichen Perlenextrakts a und des säurelöslichen Perlenextrakts b;
• 5) Gefriertrocknen des wasserlöslichen Perlenextrakts a und des säurelöslichen Perlenextrakts b nach der Entsalzung und jeweils Erhalt eines wasserlöslichen Perlenproteins und eines säurelöslichen Perlenproteins.

2. Verfahren zur Herstellung von Perlenprotein nach Anspruch 1, **dadurch gekennzeichnet, dass** die Korngröße des nanoskaligen Perlenpulvers 10-100 nm beträgt.

3. Verfahren zur Herstellung von Perlenprotein nach Anspruch 1, **dadurch gekennzeichnet, dass** als schwache Säure lebensmittelgeeignete Essigsäure, Zitronensäure oder Kohlensäure verwendet wird.

4. Verfahren zur Herstellung von Perlenprotein nach Anspruch 1, **dadurch gekennzeichnet, dass** für die Entsalzung eine Dialyseentsalzung verwendet wird: Die Entsalzung wird unter Verwendung eines Dialysebeutels mit einem Molekulargewicht von 3000-5000 Da in einer Umgebung von 2∼10°C über 3-5 Tage durchgeführt, wobei 1 Volumen der Probe 10-20 Volumina destilliertem Wasser entspricht und das Wasser 1-2 Mal täglich gewechselt werden muss.

5. Verfahren zur Herstellung von Perlenprotein nach Anspruch 1, **dadurch gekennzeichnet, dass** für die Entsalzung eine Chromatographie-Entsalzung verwendet wird: Verwenden von Sephadex G-15 für die Säulenfüllung, Anfang des Sammelns nach der ersten Entsalzung und Probenzugabe, Sammeln alle 1-2 Minuten, Sammeln mehrerer Proben, jeweils Hinzufügen von Ammoniumsulfat in kleinen Mengen, Bestimmen, ob Sedimente vorhanden sind, Überprüfen, welche der Proben herausgesalzen sind, und Sammeln Lösungen vor diesem Zeitpunkt, die nach der Entsalzung als Proteinlösung behandelt werden sollen.

## Revendications

1. Procédé de préparation d'une protéine de perle, le procédé est **caractérisé par** les étapes suivantes consistant à :
1) broyer des perles sous forme de poudre de perle à l'échelle nanométrique ;
2) réaliser une homogénéisation à ultra haute pression sur la poudre de perle à l'échelle nanométrique en utilisant un bain d'eau déminéralisée à 40 à environ 80 °C pendant 0,2 à environ 1 h, poursuivre avec une centrifugation à basses températures de 0 à environ 8 °C pour obtenir un extrait de perle soluble dans l'eau a et des sédiments, et rincer les sédiments avec une solution d'éthanol 2 à 4 fois ;
3) dissoudre les sédiments obtenus ci-dessus dans un acide faible à des températures de 30 à environ 50 °C sous vide, agiter à une faible vitesse de 80 à 120 tr/min, et faire réagir pendant 0,5 à environ 3 h, réaliser une centrifugation à basse température de 0 à environ 8 °C, et obtenir un surnageant qui est un extrait de perle soluble dans un acide b ;
4) réaliser un dessalement respectivement de l'extrait de perle soluble dans l'eau a et de l'extrait de perle soluble dans un acide b ;
5) lyophiliser l'extrait de perle soluble dans l'eau a et l'extrait de perle soluble dans un acide b après le dessalement, et obtenir respectivement une protéine de perle soluble dans l'eau et une protéine de perle soluble dans un acide.

2. Procédé de préparation d'une protéine de perle selon la revendication 1, **caractérisé en ce qu'**une granulométrie de la poudre de perle à l'échelle nanométrique est de 10 à environ 100 nm.

3. Procédé de préparation d'une protéine de perle selon la revendication 1, **caractérisé en ce que** l'acide faible adopte l'acide acétique comestible, l'acide citrique ou l'acide carbonique.

4. Procédé de préparation d'une protéine de perle selon la revendication 1, **caractérisé en ce que** le dessalement adopte le dessalement par dialyse : le dessalement est réalisée en utilisant une poche de dialyse ayant un poids moléculaire de 3 000 à environ 5 000 Da dans un environnement à 2 à environ 10 °C pendant 3 à 5 jours, dans lequel 1 volume d'échantillon correspond à 10 à 20 volumes d'eau distillée et l'eau doit être changée 1 à 2 fois par jour.

5. Procédé de préparation d'une protéine de perle selon la revendication 1, **caractérisé en ce que** le dessalement adopte le dessalement par chromatographie : utiliser Sephadex G-15 pour le remplissage de la colonne, commencer la collecte après le premier dessalement et l'ajout de l'échantillon, collecter une fois toutes les 1 à 2 minutes, regrouper plusieurs échantillons, ajouter du sulfate d'ammonium en petites quantités respectivement, déterminer si des sédiments sont présents, vérifier que les échantillons ne comprennent plus de sel, et collecter les solutions avant ce stade qui doivent être traitées comme une solution de protéine après le dessalement.
